# EUROPEAN PATENT APPLICATION

(11) **EP 3 734 262 A1**
(43) Date of publication of application: **04.11.2020**
(21) Application number: 18897062.8
(22) Date of filing: 15.10.2018
(51) Int. Cl.: G01N 23/222

(54) **DEVICE FOR ACTIVELY MONITORING FISSILE MATERIALS**

(30) Priority: 29.12.2017 WO PCT/RU2017/001019
(71) Applicant: State Atomic Energy Corporation "Rosatom" on Behalf of The Russian Federation, Moscow 119017 (RU); Joint Stock Company "Scientific-Research Institute of Chemical Technology", Moscow, 115409 (RU)
(72) Inventor: KALENOVA, Maya Yuryevna, Moscow, 109548 (RU); ANANYEV, Alexey Vladilenovich, Moscow, 117098 (RU); BASKOV, Petr Borisovich, Moscow, 123181 (RU); SKLYAROV, Sergey Vyacheslavovich, Kirovo-Chepetsk, 613045 (RU)
(74) Representative: Friese Goeden Patentanwälte PartGmbB
(86) International application number: PCT/RU2018/000683
(87) International publication number: WO 2019/132716

(57) **Abstract**

The invention relates to the field of technology concerned with the development of methods and instruments for detecting radioactive substances. The structure of the proposed invention is comprised of two coaxially arranged cylinders: an inner cylinder made of lead, which acts both as a gamma shield and as a neutron multiplier; and an outer cylinder made of polyethylene, which acts as a neutron thermalizer. Fifteen helium-3 counters with cadmium filters are equidistantly built into the wall of the outer cylinder in a circle, parallel to the generatrix. An isotropic deuterium-tritium 14 MeV neutron generator is mounted in the wall of the outer cylinder, perpendicular to the generatrix. A pedestal capable of vertical axial movement is disposed in the lower part of the inner cylinder. Prior to loading into the apparatus, a vessel and a structural material contained therein are subjected to gamma scanning. Then, by means of the movable pedestal, the vessel is mounted in the inner cylinder such that the center of mass of the structural material is situated opposite detectors. The proposed device provides high-accuracy, high-speed detection of fissile materials.

## Description

The present invention relates to the field of technology concerning the development of methods and devices for detecting radioactive substances, in particular, to the development of an installation for express detection of fissile materials (FM) and is used for a non-destructive inspection of the content of fissile materials in a metal matrix in the utilization of structural materials (SM) of spent heat-producing assemblies (SHPA).

A device for the operative non-destructive inspection of the content of fissile materials in structural materials is an analytical instrument based on the method of active neutron control. In the developed technology of reprocessing of SNUP SNF one of the key measured parameters is the content of FM in the destructured structural elements of the SHPA. Thus, at an FM content in SM above 0.001 wt. % the provision of the additional operation of the extraction of actinides is required. Characteristic features of SM of SHPAs are the gamma-background from induced radioactivity, contributing a significant error to measurements by conservative radiometric methods, and a non-uniform arrangement of a material in holding vessels, also adversely affecting the accuracy of measurement. Thus, in order to make decisions about sending SM of SHPAs to a post-purification or to a utilization, it is necessary to have a reliable method of an operative control of the content of FM in concentrations of 0.001 mass. %, which is insensitive to gamma-background and the spatial arrangement of the material. The technical solutions available today mainly are aimed not for a technological application, but to solve problems in the fields of ecological control and the restriction of the illegal transshipment of nuclear materials.

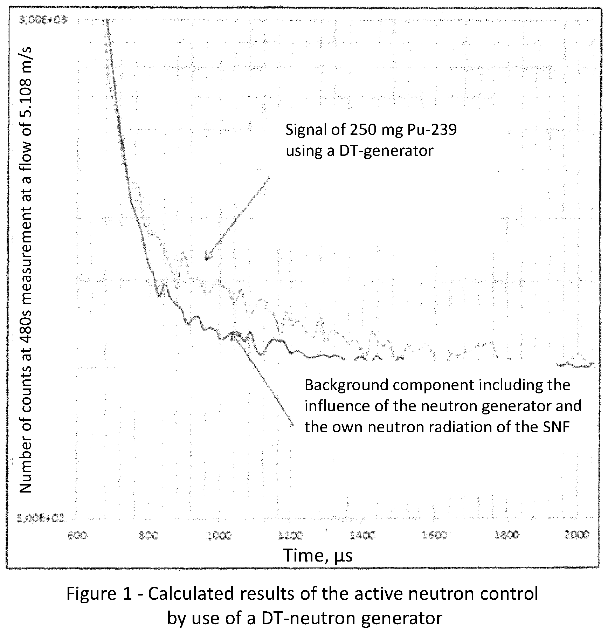

The authors of RU2589269 (IPC G01N 23/05) have proposed a device for detecting a nuclear material. It includes: a source of 2.5 MeV neutrons produced as a result of a D-D reaction, a detection unit and a processing unit. The device is intended for the detection of the content of nuclear materials (uranium -235 and plutonium -239) in closed containers and such covered with shielding materials (iron) using a movable platform (truck). The essence of the detection of the nuclear material consists in the following: the pulsed source of 2.5 MeV neutrons is located on one car, the detection unit and the processing unit on another one. The automobiles stop near the container to be inspected so that the neutron source and the detection unit are positioned adjacent to the container. Then the neutron source is switched on and a neutron measurement is carried out in a pulse mode with the help of the detection unit. The result of device operation is a detection of 1 kg of uranium -235 per 10 min of measurement at a neutron generator frequency of 10 Hz. The disadvantage of this installation is: the lack of a protection of the detection unit from gamma-irradiation; the high value of the minimum detectable mass of the fissile material (FM) due to the use of a DD generator as the source of neutrons, which provides an extremely low level of the signal Pu -239 compared with the own neutron background (Fig. 1).

Patent RU2349906 (IPC G01N 23/06) discloses a method of detecting small quantities of special nuclear materials hidden in bags, passenger luggage, cargo and transportation means. The method is based on the use of a system of two-energy radiography for scanning a transport container. As a result, when gamma-ray quanta pass, their different attenuation occurs depending on the atomic number Z of a material. Thus, the proposed method makes it possible to determine and localize within the object regions containing a substance with a high atomic number and a high density. A deficiency of the control method in question for its application to solve the present object is the impossibility to separate the response of actinides from other substances with high atomic number Z, and also the presence of iron atoms in the structural material (SM) and the holding vessel. Thus, the detection of small amounts of FM in the presence of SM is difficult.

There is known a method for detecting fissile materials (Application N° 94020227 from 01.06.1994, IPC G01N 23/222, publ. 10.04.1996) by irradiating appropriate sections of soil or a transported through customs inspection stations baggage with a pulsed or intermittent flow of fast neutrons (with an energy of 1 MeV and more) or thermal neutrons and registering delayed neutrons formed due to a forced fission by neutron counters. In order to detect uranium -235, plutonium -239, a fission process caused by thermal neutrons is used, whose cross-sections are equal to those of said elements, i.e. 586 and 748 barn, and for detecting uranium -238 and plutonium -238, the irradiation must be carried out by a direct flux of fast neutrons. The sensitivity of the method with respect to plutonium -239 reaches 0.0001%. The disadvantage of the method is that it is not intended to be used in the presence of the powerful gamma-sources Co -60 and Mn -54, which are characteristic for SM of SHPAs.

An installation is known (Ye.P. Bogolubov, S.A. Korotkov, L.A. Korytko, Method and system based on pulsed neutron generator for fissile material detection in luggage, Nuclear Instruments and Methods in Physics Research, 2004), for the control of FM in air luggage of passengers. In said document, various methods for controlling an unauthorized transport of FM are also described. For the solution of the posed problem the method of differential attenuation is chosen. The main element of the installation is a measuring chamber made of organic glass, into which the object to be investigated is placed. In the bottom of the chamber there is embedded an ING. Detectors (He -3 counters) are located outside the chamber, which register neutrons of fission. Inside detectors controlling the field of thermal neutrons in the volume are located (due to their presence, it is possible to detect neutron absorbing materials in the baggage). Signals from detectors are fed to a time analyzer and further processed on a computer. As a result, this installation is capable of detecting 5 g of ²³⁵U per 5 s. The installation's disadvantages include the small size of the measuring chamber and the absence of a structural adaptation to a high gamma-background caused by the induced activity of SM.

Separately, installations of the active control are to be considered in which as neutron sources different fissile radionuclides are used. Such installations have been developed both by foreign and by domestic companies. For example, in an Active Well Coincidence Counter (AWCC), two AmLi sources are used as the radiation source. A neutron coincidence technique is used both in active as well in passive modes. In this system, in the active mode, the minimum detectable mass of ²³⁵U is of the order of 1 g. As disadvantage of these systems the use of a large number of detectors and a small dimension of the measuring chamber can be mentioned.

The closest analog to the claimed invention is the installation of active neutron control of the mass of FM in a container (Fissile materials detection via neutron differential die-away technique, International Journal of Modern Physics, V.F. BATYAEV, O.V. BOCHKAREV, S.V. SKLYAROV. Vol 27, pp. 1460130-1-1460130-8, Singapore (2014)). The proposed by the authors installation of active neutron control is a polyethylene vessel in the form of a parallelepiped with 8 helium -3 counters with cadmium filters integrated in its wall. As activator serves a deuterium-tritium source of neutrons ING-07T. As constructive disadvantage has turned out the dependence of the efficiency of the signal detection from the location of a fissile material in the volume of the measurement vessel and the significant error introduced by the gamma-background from the induced activity of the isotopes Co -60 and Mn -54.

The inventors of the present invention were confronted with the task to develop an installation for determining small amounts (0.001 mass %) FM in SM of SHPA which is functioning under conditions of a high-power gamma-background and which is insensitive to the spatial arrangement of SM in a receiving vessel.

The technical result provided by the above set of features is a high (compared to the method of passive neutron control) accuracy and speed of determination of the FM due to the 12-fold increase of the signal level above the three-fold error of the background, the possibility of a direct unambiguous determination of the content of plutonium -239 and an indirect determination of the other actinides at a known ratio Pu : Am, as well as a non-sensitivity of the results of measurement towards the spatial arrangement of sample and gamma-irradiation. In addition, the installation can operate in a passive mode, i.e., determine the content of Cm. Consequently, knowing the content of Cm -242 and Cm -244, as well as Pu -239, it is possible to find the ratio of M_{Pu239}/(M_{Cm242} + M_{Cm244}), which in its turn makes it possible to reliably estimate the masses of any other actinides included in the SM of SHPA and heat producing elements.

The technical result is achieved in that the basis of the design of the proposed invention is two coaxially arranged cylinders: an inner one of lead, serving at the same time as gamma radiation protection and a neutron breeder, and an outer one from polyethylene serving as a neutron thermolysator. Fifteen helium -3 counters with cadmium filters are equidistantly installed in the wall of the outer cylinder in a circle parallel to the generatrix. A pulsed isotropic deuterium-tritium 14 MeV neutron generator is mounted perpendicular to the generatrix in the wall of the outer cylinder.

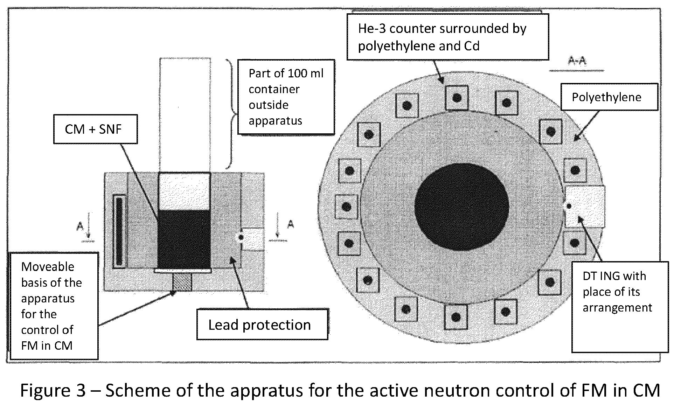

In the lower part of the inner cylinder there is a pedestal, with the possibility of its vertical displacement along the axis. The scheme of the apparatus is shown in FIG. 3. Before being loaded into the installation, the vessel with SM is subjected to gamma scanning in order to establish a center of mass. Then, by means of the movable pedestal, the vessel is placed in the inner cylinder in such a manner that the center of mass of the SM is located opposite the detectors.

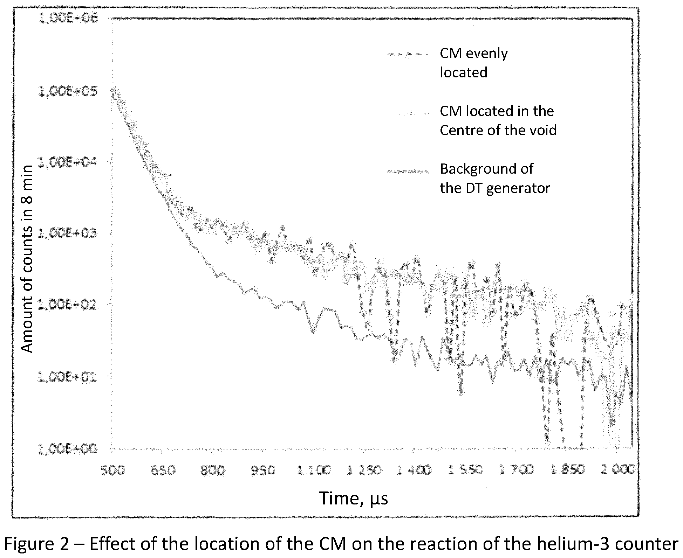

The technical solution of the invention is based on a method of differential attenuation of neutrons using a combination of Helium -3 counters with cadmium filters, a pulsed 14 MeV deuterium-tritium neutron generator, and a lead protection against gamma radiation serving as a neutron breeder at the same time. Preliminary gamma-scanning of a receiving vessel and a mobile pedestal make it possible to achieve a high efficiency of signal registration due to accurately positioning the mass center of CM between the neutron generator and the detectors. The effect of the position of the FM in the receiving vessel on the response of the helium -3 counters is presented in FIG. 2.

## Claims

1. A device for the operational non-destructive investigation of low concentrations of fissile materials in closed vessels based on helium -3 counters with cadmium filters and a pulsed neutron generator, installed in a polyethylene container, **characterized in that** the device has the form of two coaxially arranged cylinders, the inner one being from lead, which at the same time serves as gamma radiation protection and as a neutron breeder, the outer one being from polyethylene and serving as a neutron moderator, wherein in the lower part of the inner cylinder a pedestal is mounted with the possibility of its vertical movement along an axis, which positions the preliminarily gamma-scanned vessel with the SM in the inner cylinder in such a way that the center of mass of the SM is located opposite the detectors.

2. The device according to claim 1, **characterized in that** fifteen helium -3 counters with cadmium filters are equidistantly installed in the wall of the outer cylinder on a circle parallel to the generatrix.
